Europäisches Patentamt

European Patent Office

Office européen des brevets

(19)

(11) Veröffentlichungsnummer : **0 194 409**
**B1**

(12) **EUROPÄISCHE PATENTSCHRIFT**

(45) Veröffentlichungstag der Patentschrift :
28.06.89

(51) Int. Cl.⁴ : **A 61 B 17/58**

(21) Anmeldenummer : 86100320.0

(22) Anmeldetag : 11.01.86

(54) Befestigungselement für die Osteosynthes.

(30) Priorität : 11.03.85 DE 3508567

(43) Veröffentlichungstag der Anmeldung :
17.09.86 Patentblatt 86/38

(45) Bekanntmachung des Hinweises auf die Patenterteilung : 28.06.89 Patentblatt 89/26

(84) Benannte Vertragsstaaten :
AT BE CH FR GB IT LI NL SE

(56) Entgegenhaltungen :
EP--A-- 0 084 089
WO--A--85 /045 68
DE--A-- 2 727 715
DE--B-- 1 292 946
DE--B-- 1 965 350
DE--B-- 2 046 102
FR--A-- 2 529 970
GB--A-- 2 084 468
US--A-- 2 381 050
US--A-- 2 490 364

(73) Patentinhaber : fischerwerke Artur Fischer GmbH &
Co. KG
Weinhalde 14-18
D-7244 Tumlingen/Waldachtal 3 (DE)

(72) Erfinder : Fischer, Artur, Dr. h. c.
Weinhalde 34
D-7244 Waldachtal 3/Tumlingen (DE)
Erfinder : Kramer, Wolfgang, Dr. med.
Feldbergstrasse 10
D-7031 Oberjettingen (DE)

**Beschreibung**

Die Erfindung betrifft ein Befestigungselement für die Osteosynthese, insbesondere für die Fixation von Knochenfragmenten über eine Abstützplatte. Ein Befestigungselement dieser Gattung ist aus US-A-2 490 364 bekannt.

Je nach Art der Knochenfraktur werden zur Fixierung der Knochenfragmente Zugschrauben unmittelbar oder in Verbindung mit Abstützplatten verwendet. In Abhängigkeit vom Einsatzort werden Kortikalis- oder Spongiosaschrauben verwendet, die sich in ihrer Gewindeform unterscheiden. Die Spongiosaschrauben weisen ein sehr ausgeprägtes tiefes Gewinde auf, um in dem sehr weichen Knochengewebe einen ausreichenden Halt zu erzielen. Nachteilig bei diesen Schrauben ist allerdings, daß sie nach der Heilung der Fraktur nur sehr schwer insbesondere aus der Kortikalis ausschraubbar sind. Als weiterer Nachteil bei der direkten Knochenverschraubung kommt hinzu, daß die Verbindung der Schraube mit dem Knochen unelastisch ist. Kurzzeitige Überlastungen des Knochens führen zu Schädigungen der Verbindung, die zu einer Lockerung, gegebenenfalls sogar zu einem Ausbruch der Schrauben führen können.

Der Erfindung liegt die Aufgabe zugrunde, ein Befestigungselement für die Osteosynthese zu schaffen, das hohe Haltewerte ermöglicht, Überlastungen elastisch auffangen kann und leicht demontierbar ist.

Erfindungsgemäß wird dies dadurch erreicht, daß das Befestigungselement aus einer mit einem Rand versehenen Dübelhülse und einer in die Dübelhülse eingreifenden Schraube besteht, wobei das hintere Teilstück der Innenbohrung der Dübelhülse im Durchmesser geringfügig kleiner als der Gewindeaußendurchmesser der Schraube, und der Durchmesser des vorderen Teilstückes der Innenbohrung kleiner als der Kerndurchmesser der Schraube ist und die Innenbohrung der Dübelhülse im vorderen Stirnbereich auf den Durchmesser des hinteren Teilstückes der Innenbohrung vergrößert ist sowie daß ein Teil der Außenwandung der Dübelhülse mit Längsrippen versehen ist.

Für das Setzen des Befestigungselementes wird in den Knochen eine dem Außendurchmesser der Dübelhülse entsprechende Bohrung eingebracht. In diese Bohrung wird die Dübelhülse soweit eingesetzt, bis der Rand an der Außenfläche der Kortikalis anliegt. Zur Festsetzung einer Abstützplatte oder zur direkten Fixierung eines Knochenfragmentes wird die Schraube in die Dübelhülse eingedreht. Beim Eindrehen schneiden sich die Gewindespitzen der Schraube bereits im hinteren Teilstück der Innenbohrung leicht in die Innenwandung der aus einem gewebeverträglichen Kunststoff bestehenden Dübelhülse ein, ohne jedoch eine Aufweitung zu bewirken. Die Verankerung erfolgt erst im vorderen, engeren Teilstück der ungeschlitzten Dübelhülse. Durch das Gewinde der Schraube wird die Außenwandung der Dübelhülse mit mehreren Wulsten ausgebeult, so daß eine Festsetzung der Dübelhülse in der Spongiosa erreicht wird. Über den Schraubenkopf werden die Knochenfragmente bzw. die Abstützplatte verspannt.

Zur Demontage des Befestigungselementes wird die Schraube soweit ausgedreht, bis sie sich nur noch im hinteren, nicht aufgeweiteten Teilstück der Dübelhülse befindet. Über die Verbindung der in der Wandung dieses Teilstücks der Innenbohrung eingeschnittenen Gewindespitzen kann nun die Dübelhülse durch Ziehen an der Schraube aus der Bohrung entfernt werden. Wurde eine Abstützplatte verwendet, ist zunächst die Abstützplatte loszuschrauben und zu entfernen und danach zum Ausziehen der Dübelhülse die Schraube wieder in das hintere Teilstück der Innenbohrung einzudrehen.

In einer weiteren Ausgestaltung der Erfindung kann die Innenbohrung der Dübelhülse im vorderen Stirnbereich auf den Durchmesser des hinteren Teilstückes der Innenbohrung vergrößert sein. Beim Eindrehen der Schraube in die Dübelhülse wird Material nach vorne verdrängt, was unter Umständen zu einem Aufreißen des vorderen Stirnbereichs führt. Durch die Vergrößerung der Innenbohrung im vorderen Stirnbereich wird das Aufreißen der Dübelhülse vermieden.

In weiterer Ergänzung der Erfindung kann ein Teil der Außenwandung der Dübelhülse mit Längsrippen versehen sein. Mit diesen Längsrippen wird zum einen der Formschluß nach der Aufweitung der Dübelhülse und zum anderen die Drehsicherung verbessert. Bei schraubenlinienförmig angeordneten Längsrippen wird die Dübelhülse ähnlich einer Gewindebuchse eingedreht, wobei ein vorgeschnittenes Gewinde mit einem entsprechenden Gewindebohrer zweckmäßig ist.

Schließlich kann in weiterer Ausgestaltung der Erfindung auf die Außenwandung der Dübelhülse im Bereich des hinteren Teilstückes der Innenbohrung ein Metallmantel aufgebracht sein. Die beispielsweise aus einem ultrahochmolekularen Polyäthylen bestehende Dübelhülse ist im Röntgenbild nicht erfaßbar. Um den Sitz der Dübelhülse besser kontrollieren zu können bzw. um die Dübelhülse aufzufinden dient der aus einem Edelstahl bestehende Metallmantel, der entweder in Form einer Hülse oder durch Aufdampfen auf den nicht aufweitenden Teil der Dübelhülse aufgebracht ist.

In der Zeichnung sind Ausführungsbeispiele der Erfindung dargestellt.

Es zeigen :

Figur 1 das erfindungsgemäße Befestigungselement im Schnitt

Figur 2 das im Knochen verankerte Befestigungselement.

Das Befestigungselement besteht aus einer Edelstahlschraube 1 und einer Dübelhülse 2, die aus einem gewebeverträglichen Kunststoff, beispielsweise einem ultrahochmolekularen Polyäthylen hergestellt ist. Als Einschubbegrenzung dient der an der Dübelhülse 2 angeordnete Rand 3, der an der Außenfläche der Kortikalis zur Anlage kommt. Das hintere Teilstück 4a der Innenbohrung 4 weist einen Durchmesser auf, der etwas geringer ist als der Gewindeaußendurchmesser der Schraube 1. Damit schneiden sich die Gewindespitzen beim Eindrehen der Schraube geringfügig in die Innenwandung der Dübelhülse ein, ohne jedoch eine Aufweitung in diesem Bereich zu bewirken. Nach diesem Teilstück verjüngt sich die Innenbohrung 4 auf einen Durchmesser, der kleiner ist als der Kerndurchmesser der Schraube. In diesem Bereich findet die Aufweitung des Dübels statt, die für die Verankerung der Dübelhülse 2 im Knochengewebe sorgt. Zur Verbesserung des Formschlusses und als Drehsicherung sind in diesem Bereich Längsrippen 5 vorgesehen, die schraubenlinienförmig angeordnet sein können. Im vorderen Stirnbereich 4b der Dübelhülse ist die Innenbohrung wieder auf den Durchmesser des hinteren Teilstückes der Innenbohrung vergrößert, um ein Aufreißen der Dübelhülse zu verhindern. Auf den sich unmittelbar an den Rand 3 anschließenden Abschnitt der Dübelhülse 2 ist ein aus Edelstahl bestehender Metallmantel 6 aufgebracht, der eine Erfassung des Dübels im Röntgenbild ermöglicht. Der Metallmantel 6 wird entweder durch eine aufgeschobene Metallhülse oder durch eine aufgedampfte Metallschicht erreicht.

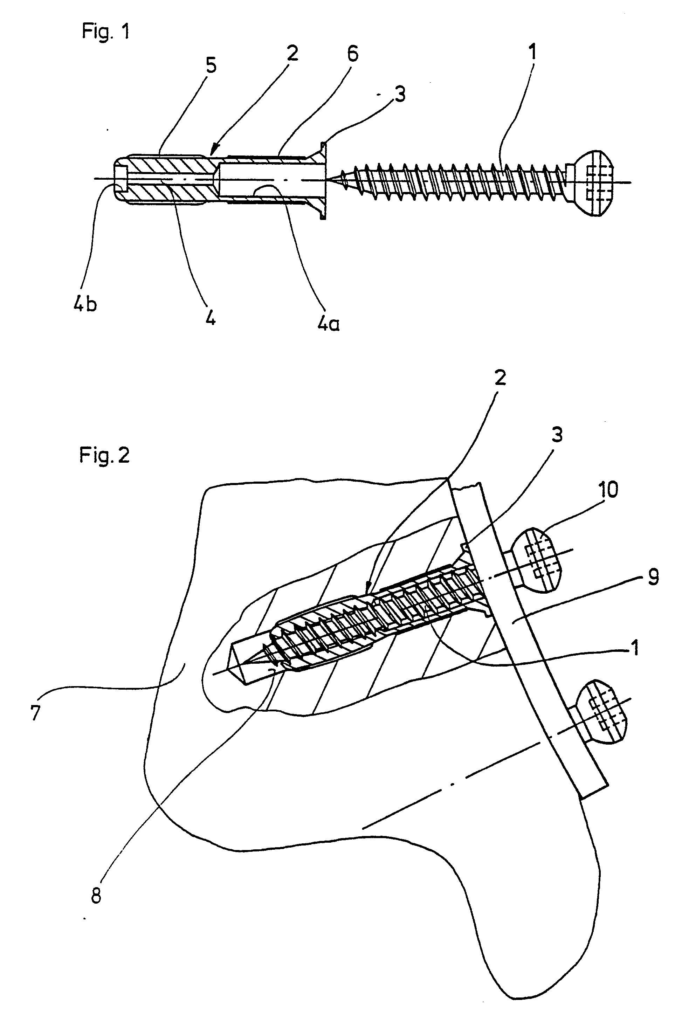

Die Darstellung nach Figur 2 zeigt das im Knochen 7 eingesetzte und verankerte Befestigungselement. Zum Setzen der Befestigungselemente werden zunächst die Bohrungen 8 erstellt. Danach wird die Dübelhülse 2 in die Bohrung 8 soweit eingedrückt, bis der Rand 3 an der Kortikalis anliegt. Nach Aufsetzen der Abstützplatte 9 wird die Schraube 1 durch die Bohrung der Abstützplatte 9 hindurch in die Innenbohrung 4 der Dübelhülse 2 eingedreht. Im Bereich der sich verengenden Innenbohrung erfolgt eine Aufweitung der Dübelhülse, die zu der Verankerung in der Spongiosa führt. Mit dem Kopf 10 der Schraube 1 wird die Abstützplatte gegen den Knochen verspannt.

Nach Heilung der Fraktur werden die Abstützplatte 9 und die Befestigungselemente wieder entfernt. Zu diesem Zweck werden die Schrauben 1 herausgedreht, so daß die Abstützplatte abgenommen werden kann. Danach wird die Schraube wieder in das hintere Teilstück 4a der Innenbohrung eingedreht und mit der Schraube 1 die Dübelhülse 2 aus dem Bohrloch 8 gezogen.

## Patentansprüche

1. Befestigungselement für die Osteosynthese, insbesondere für die Fixation von Knochenfragmenten über eine Abstützplatte, dadurch gekennzeichnet, daß das Befestigungselement aus einer mit einem Rand versehenen Dübelhülse und einer in die Dübelhülse eingreifenden Schraube besteht, wobei das hintere Teilstück der Innenbohrung der Dübelhülse im Durchmesser geringfügig kleiner als der Gewindeaußendurchmesser der Schraube, und der Durchmesser des vorderen Teilstückes der Innenbohrung kleiner als der Kerndurchmesser der Schraube ist und die Innenbohrung der Dübelhülse im vorderen Stirnbereich auf den Durchmesser des hinteren Teilstückes der Innenbohrung vergrößert ist sowie, daß ein Teil der Außenwandung der Dübelhülse mit Längsrippen versehen ist.

2. Befestigungselement nach Anspruch 1, dadurch gekennzeichnet, daß die Längsrippen schraubenlinienförmig angeordnet sind.

3. Befestigungselement nach Anspruch 1, dadurch gekennzeichnet, daß auf die Außenwandung der Dübelhülse im Bereich des hinteren Teilstückes der Innenbohrung ein Metallmantel aufgebracht ist.

## Claims

1. A fixing element for osteosynthesis, especially for setting bone fragments using a support plate, characterized in that the fixing element comprises a plug sleeve provided with a rim, and a screw engaging in the plug sleeve, wherein the rear portion of the internal bore of the plug sleeve is slightly smaller in diameter than the external diameter of the thread of the screw, and the diameter of the leading portion of the internal bore is smaller than the core diameter of the screw, and the internal bore of the plug sleeve is, in the region of the leading end, enlarged to the diameter of the rear portion of the internal bore, and in that part of the outer wall of the plug sleeve is provided with longitudinal fins.

2. A fixing element according to claim 1, characterized in that the longitudinal fins are arranged helically.

3. A fixing element according to claim 1, characterized in that a metal covering is applied to the external wall of the plug sleeve in the region of the rear portion of the internal bore.

## Revendications

1. Elément de fixation pour l'ostéosynthèse, en particulier pour la fixation de fragments d'os par l'intermédiaire d'une plaque de soutien, caractérisé en ce que l'élément de fixation est constitué par une douille formant cheville munie d'un rebord et par une vis venant en prise dans la douille formant cheville, la partie arrière de l'alésage intérieur de la douille formant cheville étant d'un diamètre très légèrement plus petit que le diamètre extérieur du filetage de la vis, et le diamètre de la partie avant de l'alésage intérieur étant plus petit que le diamètre à fond de filet de la vis, et l'alésage intérieur de la douille s'agrandissant dans la zone frontale avant jusqu'à la dimension du diamètre de la partie arrière de l'alésage intérieur, et en ce qu'une partie de la paroi extérieure de la douille formant cheville est munie de nervures longitudinales.

2. Elément de fixation selon la revendication 1, caractérisé en ce que les nervures longitudinales sont disposées selon une forme hélicoïdale.

3. Elément de fixation selon la revendication 1, caractérisé en ce qu'une enveloppe métallique est rapportée sur la paroi extérieure de la douille formant cheville dans la zone de la partie arrière de l'alésage intérieur.

Fig. 1

Fig. 2